# EUROPÄISCHE PATENTSCHRIFT

(11) **EP 2 823 031 B1**
(45) Veröffentlichungstag und Bekanntmachung des Hinweises auf die Patenterteilung: **11.04.2018**
(21) Anmeldenummer: 13707627.9
(22) Anmeldetag: 05.03.2013
(51) Int. Cl.: C12M 1/107, C12M 1/16

(54) **BIOREAKTOR ZUR METHANISIERUNG VON BIOMASSE SOWIE EIN VERFAHREN ZUM BETREIBEN EINES SOLCHEN BIOREAKTORS**
BIOREACTOR FOR METHANIZING BIOMASS AND METHOD FOR OPERATING SUCH A BIOREACTOR
BIORÉACTEUR POUR LA MÉTHANISATION DE BIOMASSE ET PROCÉDÉ SERVANT À FAIRE FONCTIONNER UN BIORÉACTEUR DE CE TYPE

(30) Priorität: 06.03.2012 DE 202012100788 U; 07.03.2012 DE 202012100816 U
(43) Veröffentlichungstag der Anmeldung: 14.01.2015
(73) Patentinhaber: BEKON GmbH, 85774 Unterföhring (DE)
(72) Erfinder: LUTZ, Peter, 81925 München (DE); LIEBENEINER, Rolf, 82131 Stockdorf (DE)
(74) Vertreter: Schober, Mirko
(86) Internationale Anmeldenummer: PCT/EP2013/054339
(87) Internationale Veröffentlichungsnummer: WO 2013/131876

(56) Entgegenhaltungen:
- WO-A1-2007/028642
- WO-A2-2005/054423
- DE-U1-202005 019 132

## Beschreibung

Die Erfindung betrifft einen Bioreaktor zur Methanisierung von Biomasse nach dem Oberbegriff des Anspruchs 1, eine Biogasanlage mit einer Mehrzahl von solchen Bioreaktoren nach Anspruch 7 sowie ein Verfahren zum Betreiben eines solchen Bioreaktors nach Anspruch 8.

Ein Bioreaktor der hier vorgestellten Art ist aus der EP 1 301 583 B1 bekannt. Hinsichtlich der konstruktiven Ausgestaltung wird auf die Offenbarung der EP 1 301 583 B1 vollinhaltlich Bezug genommen. Dieser bekannte Bioreaktor ist nach Art einer Fertiggarage ausgebildet und weist eine Front- und eine Rückseite auf. Die komplette Frontseite ist offen und wird durch ein klappenförmiges Tor gasdicht verschlossen. Durch das Tor kann frische Biomasse in den Bioreaktor eingebracht und verbrauchte Biomasse aus dem Bioreaktor entladen werden. Die verbrauchte Biomasse wird üblicherweise kompostiert. Nach der geltenden Bioabfallverordnung (BioabfV) darf Kompost nur auf Frei- und Grünflächen ausgebracht werden, wenn er seuchen- und phytohygienisch unbedenklich ist, d. h., dass Unkrautsamen und phytopatogene Keime wie z. B. Feuerbranderreger müssen abgetötet sein. Um dies zu Gewährleisten muss die Temperatur in der Kompostrotte über eine vorgegebene Zeitdauer in einem bestimmten Temperaturbereich liegen. Dies erhöht die Anforderungen an die Sorgfalt bei der Kompostierung. Wenn bereits die Biogaserzeugung im thermophilen Bereich (Temperatur > 55°C) erfolgt, muss bei der anschließenden Kompostierung der verbrauchten Biomasse weniger sorgfältig gearbeitet werden. Allerdings besteht bei den aus der EP 1 301 583 B1 bekannten Bioreaktoren die Gefahr, dass die verbrauchte Biomasse beim Entladen wieder kontaminiert wird.

Aus WO2007/028642 ist ebenfalls diskontinuierliche Anlage bekannt, bei zum Beladen des Bioreaktors mit frischer Biomasse und zum Entladen der verbrauchten Biomasse die Biogaserzeugung unterbrochen und das Tor zum Be- und Entladen geöffnet wird.

Aus der DE202005019132U1 ist eine kontinuierliche Anlage bekannt, bei der die Zufuhr von frischer Biomasse über eine erste Förderschnecke und die Entnahme der verbrauchten Biomasse über eine zweite Förderschnecke kontinuierlichen erfolgt. Die Biogasproduktion erfolgt daher kontinuierlich.

Ausgehend von dem Bioreaktor nach der EP-B 1 301 583 oder der WO 2007/028642 A1 ist es Aufgabe der vorliegenden Erfindung eine Bioreaktor anzugeben, bei dem die Gefahr der Kontaminierung der verbrauchten Biomasse in seuchen-und phytohygienischer Hinsicht erheblich verringert ist. Weiter ist es Aufgabe der Erfindung ein Verfahren zum Betreiben eines solchen Bioreaktors anzugeben.

Die Lösung dieser Aufgabe erfolgt durch einen Bioreaktor nach Anspruch 1.

Dadurch, dass der langgestreckte Reaktorbehälter ein befahrbares Belade-Tor und ein befahrbares Entlade-Tor umfasst, die an gegenüberliegenden Enden des langgestreckten Reaktorbehälters angeordnet sind, ist es möglich, die durch thermophile Prozessführung bei der Vergärung seuchen- und phytohygienisch unbedenkliche verbrauchte Biomasse über das Entlade-Tor aus dem Reaktorbehälter zu entnehmen und unmittelbar der Kompostierung zuzuführen. Der Bioreaktor besitzt damit eine "sauberes" Entlade-Tor und ein "unsauberes" Belade-Tor. Unerwünschte Keime, Samen und Bakterien aus dem Bereich des Belad-Tores können somit nicht in den "sauberen" Bereich des Entlade-Tors gelangen. Durch den Bioreaktor mit zwei Toren ist es möglich eine "saubere", d. h. eine seuchen- und phytohygienisch unbedenkliche Entlade-Zone von der seuchen- und phytohygienisch nicht unbedenklichen Belade-Zone räumlich zu trennen. Durch den ebenerdigen und befahrbaren Boden des Reaktorbehälters vereinfacht sich die Be- und Entladung des Reaktorbehälters.

Bei thermophiler Prozessführung bei der Biogaserzeugung erfolgt die Biogaserzeugung mit Bakterien, die sich in einem Temperaturbereich von etwa 50°C bis 60°C optimal "arbeiten", während bei mesophiler Prozessführung die Biogaserzeugung durch Bakterienstämme erfolgt, die im Bereich von etwa 30°C und 35°C optimal "arbeiten". Bei thermophiler Prozessführung bei der Biogaserzeugung ist daher bereits die verbrauchte Biomasse seuchen- und phytohygienisch unbedenklich.- Anspruch 9 - und unmittelbar folgende Kompostierung kann mit geringerer Sorgfalt ausgeführt werden- Anspruch 10.

Durch die den gesamten Querschnitt einnehmenden Tore vereinfacht sich die Be- und Entladung des Reaktorbehälters weiter - Anspruch 2.

Durch die Konstruktion des Reaktorbehälters nach Art einer Fertiggarage verringern sich die Herstellungskosten für den Reaktorbehälter - Anspruch 3.

Durch die hydraulische Betätigung der beiden Tore und deren Ausbildung als Klappen vereinfacht sich das Be- und Entladen des Reaktorbehälters - Ansprüche 4 und 5.

Durch die Rückhaltevorrichtungen hinter den Toren werden die Tore druckentlastet - Anspruch 6.

Durch die Mehrzahl von Biogasreaktoren ist eine quasi kontinuierliche Biogaserzeugung im Batch-Betrieb möglich - Anspruch 7.

Weitere Einzelheiten, Merkmale und Vorteile der Erfindung ergeben sich aus den Unteransprüchen und der nachfolgenden Beschreibung bevorzugter Ausführungsformen. Hierzu zeigt:

Es zeigt:
Fig. 1 schematisch Schnittdarstellung des erfindungsgemäßen Bioreaktors mit zwei Toren;
Fig. 2 eine perspektivische Ansicht des Biorektors nach Fig. 1 von vorne oder von hinten mit geöffnetem Belade-Tor bzw. Entlade-Tor; und
Fig. 3 eine Querschnittsdarstellung durch den Bioreaktor mit den Rohrleitungen der Wand- und Fußbodenheizung.

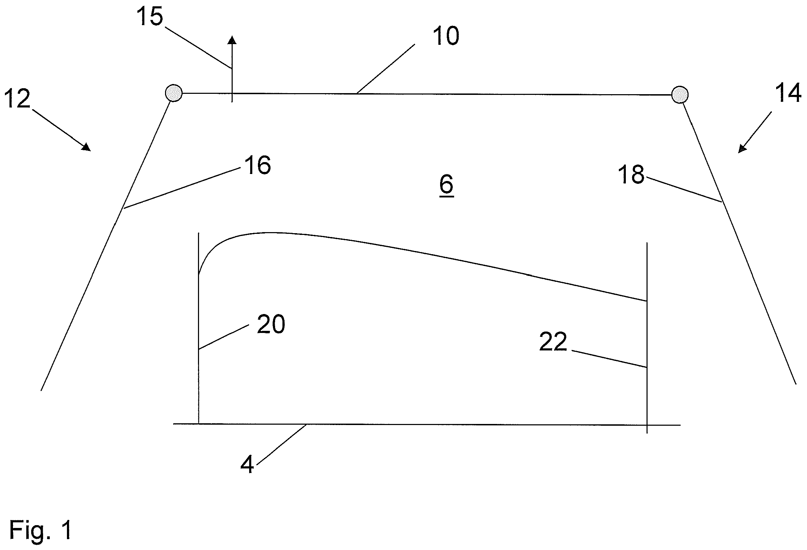

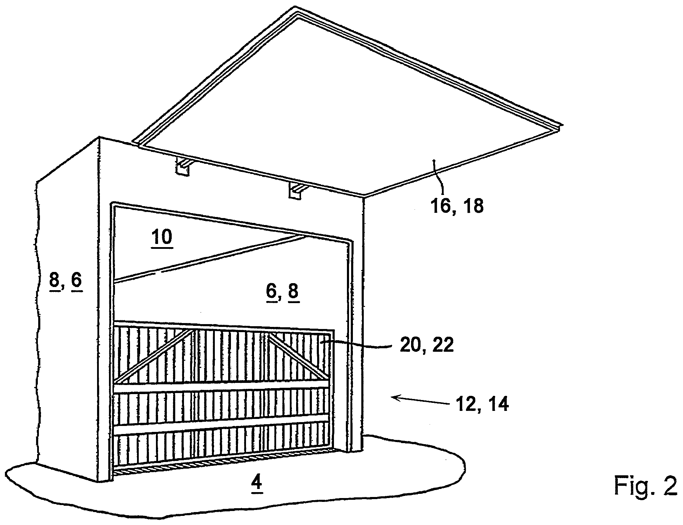

Ein Bioreaktor bzw. Biogasreaktor nach den Figuren 1 und 2 umfasst einen quaderförmigen langgestreckten Reaktorbehälter 2, der nach Art einer Fertiggarage aus Stahlbeton besteht und sechs plane Wandelemente umfasst, nämlich eine Bodenplatte 4, zwei Seitenwände 6 und 8, eine Deckplatte 10 und eine offene Vorderseite 12, eine offene Rückseite 14 und einen Biogasentnahmeanschluss 15. Die offene Vorderseite ist 12 ist durch eine erstes Tor 16 - Belade-Tor - und die offen Rückseite 14 ist durch ein zweites Tor 18 - Entlade-Tor - gasdicht verschließbar. Beide Tore 15, 18 sind hydraulisch betätigbar. Hinsichtlich der Ausgestaltung der beiden Tore wird vollinhaltlich auf die WO2007/028642A1 Bezug genommen.

Fig. 2 zeigt perspektivisch die Vorderseite 12 des Reaktorbehälters 2 mit geöffnetem Belade-Tor 16. Im Reaktorbehälter 2 unmittelbar hinter dem Belade-Tor 16 ist eine erste Rückhaltevorrichtung 20 vorgesehen, die verhindert, dass die Biomasse unmittelbar gegen das Belade-Tor 16 drückt. Die erste Rückhaltevorrichtung 20 stellt damit eine Druckentlastung des gasdichten Belade-Tors 16 dar.

Fig. 2 kann auch als perspektivische Ansicht der Rückseite 14 des Reaktorbehälters 2 mit offenem Entlade-Tor 18 interpretiert werden. Hinter dem Entlade-Tor 18 ist eine zweite Rückhaltevorrichtung 22 vorgesehen, die ebenfalls verhindert, dass die Biomasse unmittelbar gegen das Entlade-Tor 18 drückt.

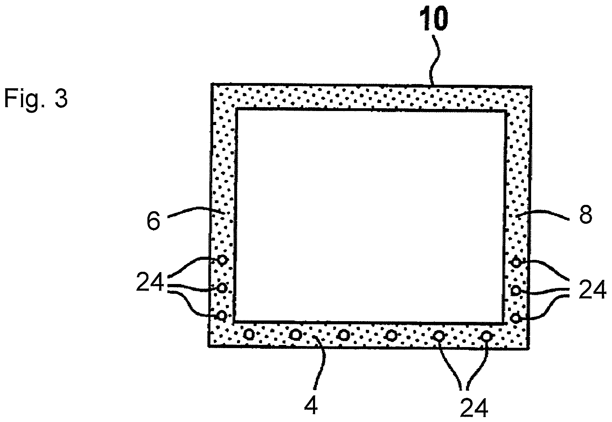

In den Seitenwänden 6, 8 und in der Bodenplatte sind Rohrschlangen 24 zur Beheizung der Biomasse in dem Reaktorbehälter 2 angeordnet. Damit kann die Biogaserzeugung in dem Reaktorbehälter thermophil erfolgen. Die Rohrschlangen 24 sind in der Schnittdarstellung quer durch den Reaktorbehälter 2 in Fig. 3 dargestellt.

Hinsichtlich der weiteren Ausgestaltung des Bioreaktors und der Biogasanlage mit einer Mehrzahl von Bioreaktoren gemäß der vorliegenden Erfindung wird vollinhaltlich auf die EP1997875B1 und die DE102008015240B4 verwiesen.

### Bezugszeichenliste

- 2: Reaktorbehälter
- 4: Bodenplatte
- 6,8: Seitenwände
- 10: Deckplatte
- 12: offene Vorderseite
- 14: offene Rückseite
- 15: Biogasentnahmeanschluss
- 16: erstes Tor - Belade-Tor
- 18: zweites Tor - Entlade-Tor
- 20: erste Rückhaltevorrichtung
- 22: zweite Rückhaltevorrichtung
- 24: Heizungsrohrschlangen

## Patentansprüche

1. Bioreaktor zur Methanisierung von Biomasse nach dem Prinzip der Trockenfermentierung, mit
- einem langgestreckten Reaktorbehälter (2) mit einem Boden (4) auf dem die Biomasse aufliegt, und der ein erstes Ende (12) und ein zweites Ende (14) aufweist,
- einem an dem ersten Ende (12) angeordneten ersten befahrbaren gasdichten Tor (16) zum Beladen des Reaktorbehälters (2) mit frischer Biomasse, und
- einem Biogasentnahmeanschluss (15),
**dadurch gekennzeichnet,**
**dass** der Boden (4) des Reaktorbehälters (2) ebenerdig ist und
**dass** an dem zweiten Ende (14) ein zweites befahrbares gasdichtes Tor (18) zum Entladen von verbrauchter Biomasse aus dem Reaktorbehälter (2) vorgesehen ist.

2. Bioreaktor nach Anspruch 1, **dadurch gekennzeichnet, dass** die beiden befahrbaren gasdichten Tore (16, 18) im Wesentlichen die gesamte Querschnittsfläche des Reaktorbehälters (2) einnehmen.

3. Bioreaktor nach einem der vorhergehenden Ansprüche, **dadurch gekennzeichnet, dass** der Reaktorbehälter (2) nach Art einer Fertiggarage aus Stahlbeton ausgebildet ist und einen rechteckigen Querschnitt aufweist.

4. Bioreaktor nach einem der vorhergehenden Ansprüche, **dadurch gekennzeichnet, dass** die beiden einander gegenüberliegenden Tore (16, 18) hydraulisch betätigbar sind.

5. Bioreaktor nach einem der vorhergehenden Ansprüche, **dadurch gekennzeichnet, dass** die beiden einander gegenüberliegenden Tore (16, 18) als Klappen ausgebildet sind und oben an dem Reaktorbehälter (2) angelenkt sind.

6. Bioreaktor nach einem der vorhergehenden Ansprüche, **dadurch gekennzeichnet, dass** die im Reaktorbehälter (2) jeweils hinter den beiden Toren (16, 18) eine erste und eine zweite Rückhaltevorrichtung (20, 22) zur Druckentlastung der beiden Tore (16, 18) vorgesehen ist.

7. Biogasanlage mit einer Mehrzahl von Biogasreaktoren nach einem der vorhergehenden Ansprüche zum Erzeugen von Biogas im Batchbetrieb.

8. Verfahren zum Betreiben eines Bioreaktors oder einer Biogasanlage nach einem der vorhergehenden Ansprüche, mit den Verfahrensschritten:
- Beladen des Reaktorbehälters (2) mit frischer Biomasse durch das erste Tor (16),
- Erzeugung von Biogas durch Methanisierung der Biomasse nach dem Prinzip der Trockenfermentation im thermophilen Bereich, und
- Entladen der keimarmen verbrauchten Biomasse durch das zweite Tor (18).

9. Verfahren nach Anspruch 9, **dadurch gekennzeichnet, dass** die verbrauchte Biomasse nach dem Entladen aus dem Reaktorbehälter (2) unmittelbar kompostiert wird.

## Claims

1. Bioreactor for methanizing biomass according to the dry fermentation principle, with
- an elongated reactor container (2) with a base (4) on which the biomass is placed, and which has a first end (12) and a second end (14)
- a first passable gas-tight gate (16) arranged at the first end (12) for loading the reactor container (2) with fresh biomass, and
- a biogas removal connector (15),
**characterised in that**
the base (4) of the reactor container (2) is at ground level and that a second passable gas-tight gate (18) is provided at the second end (14) for unloading spent biomass from the reactor container (2).

2. Bioreactor according to claim 1
**characterised in that**
the two passable gas-tight gates (16, 18) occupy substantially the entire cross-sectional surface area of the reactor container (2).

3. Bioreactor according to one of the preceding claims,
**characterised in that**
the reactor container (2) is configured in the manner of a ready-made garage of steel concrete and has a rectangular cross-section.

4. Bioreactor according to one of the preceding claims
**characterised in that**
the two mutually opposing gates (16, 18) can be hydraulically operated.

5. Bioreactor according to one of the preceding claims
**characterised in that**
the two mutually opposing gates (16, 18) are designed as flaps and are attached to the reactor container (2) at the top.

6. Bioreactor according to one of the preceding claims
**characterised in that**
a first and a second retaining device (20, 22) are provided in the reactor container (2) behind each of the two gates (16, 18) respectively in order to relieve the pressure on the two gates (16, 18).

7. Biogas plant with a plurality of biogas reactors according to one of the preceding claims for generating biogas in batch operation.

8. Method for operating a bioreactor or a biogas plant according to one of the preceding claims with the method steps:
- loading the reactor container (2) with fresh biomass through the first gate (16),
- generating biogas by methanizing the biomass according to the dry fermentation principle in the thermophilic region, and
- unloading the low-germ spent biomass through the second gate (18).

9. Method according to claim 9
**characterised in that**
the spent biomass is composted immediately after unloading from the reactor container (2).

## Revendications

1. Bioréacteur pour la méthanisation de biomasse selon le procédé de la fermentation en voie sèche, avec
- une cuve de réacteur (2) de forme allongée, qui comprend un fond (4), sur lequel repose la biomasse et qui présente une première extrémité (12) et une deuxième extrémité (14),
- une première porte étanche aux gaz (16), mobile, disposée sur la première extrémité (12), laquelle est destinée au chargement de la cuve de réacteur (2) avec de la biomasse fraîche, et
- un raccordement de prélèvement de biomasse (15),
**caractérisé en ce que**
le fond (4) de la cuve de réacteur (2) est situé au niveau du sol et que, sur la deuxième extrémité (14), est prévue une deuxième porte étanche aux gaz (18), mobile, qui est destinée au déchargement de la biomasse usée hors de la cuve de réacteur (2).

2. Bioréacteur selon la revendication 1,
**caractérisé en ce que**
les deux portes étanches aux gaz (16, 18), mobiles, occupent sensiblement la totalité de la section de coupe transversale de la cuve de réacteur (2).

3. Bioréacteur selon l'une des revendications précédentes,
**caractérisé en ce que**
la cuve de réacteur (2) est réalisée en béton armé, à la manière d'un garage préfabriqué, et présente une section transversale rectangulaire.

4. Bioréacteur selon l'une des revendications précédentes,
**caractérisé en ce que**
les deux portes (16, 18), situées à l'opposé l'une de l'autre, peuvent être actionnées hydrauliquement.

5. Bioréacteur selon l'une des revendications précédentes,
**caractérisé en ce que**
les deux portes (16, 18), situées à l'opposé l'une de l'autre, sont réalisées sous la forme d'abattants et sont articulées, en haut, sur la cuve de réacteur (2).

6. Bioréacteur selon l'une des revendications précédentes,
**caractérisé en ce que**,
dans la cuve de réacteur (2), derrière les deux portes (16, 18), sont respectivement prévues une première et une deuxième installation de retenue (20, 22), pour la décharge de pression des deux portes (16, 18).

7. Installation de biogaz avec une pluralité de bioréacteurs selon l'une des revendications précédentes, destinée à la production de biogaz selon le processus en lots.

8. Procédé de fonctionnement d'un bioréacteur ou d'une installation de production de biogaz selon l'une des revendications précédentes comprenant les étapes de processus:
- chargement de la cuve de réacteur (2) avec de la biomasse fraîche, par la première porte (16),
- production de biogaz par méthanisation de la biomasse selon le principe de la fermentation en voie sèche dans le domaine thermophile, et
- déchargement de la biomasse usée, pauvre en germes, par la deuxième porte (18).

9. Procédé selon la revendication 8,
**caractérisé en ce que**
la biomasse usée est compostée directement après avoir été déchargée de la cuve de réacteur (2).
